# EUROPEAN PATENT SPECIFICATION

(11) **EP 1 235 810 B1**
(45) Date of publication and mention of the grant of the patent: **09.07.2003**
(21) Application number: 00976715.3
(22) Date of filing: 27.10.2000
(51) Int. Cl.: C07D 231/12, C07D 231/22, C07D 231/26, C07D 231/38, C07D 401/04

(54) **PROCESS OF MAKING SUBSTITUTED PYRAZOLES**
VERFAHREN ZUR HERSTELLUNG VON SUBSTITUIERTEN PYRAZOLEN
PROCEDE DE PRODUCTION DE PYRAZOLES SUBSTITUES

(30) Priority: 29.10.1999 US 162476 P; 28.03.2000 US 192651 P
(43) Date of publication of application: 04.09.2002
(73) Proprietor: BOEHRINGER INGELHEIM PHARMACEUTICALS INC., Ridgefield, Connecticut 06877-0368 (US)
(72) Inventor: BARON, James, A., Hilliard, OH 43026 (US); FARINA, Vittorio, Wilton, CT 06897 (US); HADDAD, Nizar, Danbury, CT 06811 (US)
(74) Representative: Kompter, Hans-Michael, Dr.
(86) International application number: US0029891
(87) International publication number: WO01032627

(56) References cited:
- WO-A-99/23091
- US-A- 5 616 723
- US-A- 5 969 153
- LEE K J ET AL: "SYNTHESIS OF PYRAZOLO-FUSED HETEROCYCLES BY A TANDEM APPEL'S DEHYDRATION/ELECTROCYCLIZATION METHODOLOGY" JOURNAL OF HETEROCYCLIC CHEMISTRY,HETEROCORPORATION. PROVO,US, vol. 34, no. 6, November 1997 (1997-11), pages 1795-1799, XP000943029 ISSN: 0022-152X
- SEBLE WAGAW ET AL:: "Palladium-catalyzed Strategy for the Preparation of Indoles: A Novel Entry into the Fisher Indole Synthesis" JOURNAL OF THE AMERICAN CHEMICAL SOCIETY,AMERICAN CHEMICAL SOCIETY, WASHINGTON, DC,US, vol. 120, no. 26, 1998, pages 6621-6622, XP000983304 ISSN: 0002-7863

## Description

### Field of Invention

The present invention relates to novel synthesis of substituted aryl and heteroaryl pyrazole compounds of the formula (I) described herein.

### Background

The aryl and heteroaryl pyrazole structure is found in a large number and variety of compounds that possess important biological activities and pharmacological properties. Makino, K. et al. *J. Heterocyclic Chem.* **1998**, *35,* 489; Elguero, J. *Compr. Heterocycl. Chem. II* **1996**, *3,* 1. For example, WO 98/52558 and WO 99/23091 disclose heteroaryl urea compounds which are indicated to be useful in treating cytokine mediated diseases. U.S. Pat. No. 5,162,360 discloses N-substituted aryl-N'-heterocyclic substituted urea compounds which are described as being useful for treating hypercholesterolemia and atheroclerosis.

The synthesis of this important family of compounds is well reviewed. See Makino, K. et al. *supra,* Takagi, K. et al. *J. Heterocyclic Chem.* **1996,** 33, 1003; El-Rayyes, N. R. et al. *Synthesis* **1985,** 1028; Sammes, M. P. et al. *Advances in Heterocyclic Chemistry, Vol* 34, Academic Press, **1983;** Behr, L. C. et al. *The Chemistry of Heterocyclic Compounds,* Weissberger, A., ed., Interscience Publishers, John Wiley and Sons, **1967.** The conventional approach for pyrazole synthesis is the condensation of an aryl hydrazine with 1,3-diketones or their equivalents, such as β-ketoesters, β-cyanoketones and others. However, aryl hydrazines have not been widely available by convenient, scalable chemistry. Buchwald and Hartwig have recently described a general and practical synthesis of N-arylated benzophenone hydrazones. Buchwald, S. L. et al. *J. Am. Chem. Soc.* **1998**, *120*, 6621; Hartwig, J. F. *Angew. Chem., Int. Ed.* **1998,** 37, 2090.

Unfortunately, their hydrolysis to N-aryl hydrazines has not been demonstrated. There is therefore a clear need for a synthesis of substituted pyrazoles which overcomes limitations of well known syntheses.

### Summary of the Invention

The present invention addresses the need in the art for a versatile new synthesis of substituted pyrazoles of the formula (I): 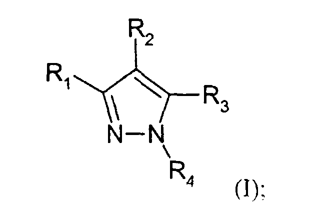 wherein R₁, R₂, R₃ and R₄ are defined herein below, by providing for the first time a process of making a variety of pyrazoles from substituted benzophenone hydrazones with different 1,3-bifunctional groups.

### Detailed Description of Preferred Embodiments

In the present invention, it was postulated that upon treatment of such hydrazones with dicarbonyl compounds or related functionalities apparent to the skilled artisan, a transhydrazonation reaction would take place^{3a}, leading eventually to pyrazole compounds of the formula (I). Such a synthesis will benefit from the demonstrated palladium catalyzed cross couplings of benzophenone hydrazone to various aryl halides and overcomes limitations associated with the availability of aryl and heteroaryl hydrazines.⁴ The novel process of the invention also provides product compounds with a desirable high regio specificity as shown in schemes 1 - 4 below.

In one embodiment of the invention there is provided the process of making a pyrazole compound of the formula(I): 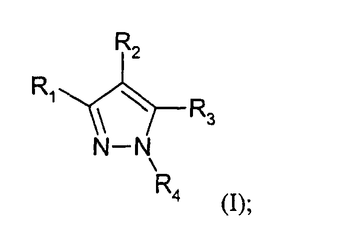 wherein R₁, R₂, R₃ and R₄ are defined as follows:
each R₁ and R₃ are independently chosen from:
   amino and C₁₋₁₀ alkyl optionally partially or fully halogenated and optionally substituted with one to three C₃₋₁₀cycloalkanyl, C₁₋₆alkoxy, phenyl, naphthyl, pyridinyl, pyrimidinyl, pyrazinyl, pyridazinyl, pyrrolyl, imidazolyl, pyrazolyl, thienyl, furyl, isoxazolyl or isothiazolyl; each of the aforementioned being optionally substituted with one to five groups chosen from halogen, C₁₋₆ alkyl which is optionally partially or fully halogenated, C₃₋₈ cycloalkanyl, C₅₋₈ cycloalkenyl and C₁₋₃ alkoxy which is optionally partially or fully halogenated; wherein both R₁ and R₃ cannot simultaneously be amino;
R₂ is chosen from:
   hydrogen, C₁₋₆ branched or unbranched alkyl optionally partially or fully halogenated and aryl optionally partially or fully halogenated;
R₄ is chosen from:
   phenyl, naphthyl, morpholinyl, pyridinyl, pyrimidinyl, pyrazinyl, pyridazinyl, pyrrolyl, pyrrolidinyl, imidazolyl, pyrazolyl, thiazolyl, oxazoyl, triazolyl, tetrazolyl, thienyl, furyl, tetrahydrofuryl, isoxazolyl, isothiazolyl, quinolinyl, isoquinolinyl, indolyl, benzimidazolyl, benzofuranyl, benzoxazolyl, benzisoxazolyl, benzpyrazolyl, benzothiofuranyl, cinnolinyl, pterindinyl, phthalazinyl, naphthypyridinyl, quinoxalinyl, quinazolinyl, purinyl and indazolyl, each of the aforementioned is optionally substituted with one to three phenyl, naphthyl, heterocycle or heteroaryl as hereinabove described in this paragraph, C₁₋₆ branched or unbranched alkyl which is optionally partially or fully halogenated, cyclopropanyl, cyclobutanyl, cyclopentanyl, cyclohexanyl, cycloheptanyl, bicyclopentanyl, bicyclohexanyl, bicycloheptanyl, phenyl C₁₋₅ alkyl, naphthyl C₁₋₅ alkyl, halogen, hydroxy, oxo, nitrile, C₁₋₃ alkoxy optionally partially or fully halogenated, phenyloxy, naphthyloxy, heteroaryloxy or heterocyclicoxy wherein the heterocyclic or heteroaryl moiety is as hereinabove described in this paragraph, nitro, phenylamino, naphthylamino, heteroaryl or heterocyclic amino wherein the heteroaryl or heterocyclic moiety is as hereinabove described in this paragraph, NH₂C(O), a mono- or di-(C₁₋₃alkyl) aminocarbonyl, C₁₋₅ alkyl-C(O)-C₁₋₄ alkyl, amino-C₁₋₅ alkyl, mono- or di-(C₁₋₃alkyl)amino-C₁₋₅ alkyl, amino-S(O)₂, di-(C₁₋₃alkyl)amino-S(O)₂, R₇-C₁₋₅ alkyl, R₈-C₁₋₅ alkoxy, R₉-C(O)-C₁₋₅ alkyl, R₁₀-C₁₋₅ alkyl(R₁₁)N or carboxy-mono- or di-(C₁₋₅alkyl)-amino;
   a fused aryl chosen from benzocyclobutanyl, indanyl, indenyl, dihydronaphthyl, tetrahydronaphthyl, benzocycloheptanyl and benzocycloheptenyl, or a fused heteroaryl chosen from cyclopentenopyridinyl, cyclohexanopyridinyl, cyclopentanopyrimidinyl, cyclohexanopyrimidinyl, cyclopentanopyrazinyl, cyclohexanopyrazinyl, cyclopentanopyridazinyl, cyclohexanopyridazinyl, cyclopentanoquinolinyl, cyclohexanoquinolinyl, cyclopentanoisoquinolinyl, cyclohexanoisoquinolinyl, cyclopentanoindolyl, cyclohexanoindolyl, cyclopentanobenzimidazolyl, cyclohexanobenzimidazolyl, cyclopentanobenzoxazolyl, cyclohexanobenzoxazolyl, cyclopentanoimidazolyl, cyclohexanoimidazolyl, cyclopentanothienyl and cyclohexanothienyl; wherein the fused aryl or fused heteroaryl ring is independently substituted with zero to three phenyl, naphthyl, pyridinyl, pyrimidinyl, pyrazinyl, pyridazinyl, pyrrolyl, imidazolyl, pyrazolyl, thienyl, furyl, isoxazolyl, isothiazolyl, C₁₋₆ alkyl which is optionally partially or fully halogenated, halogen, nitrile, C₁₋₃ alkoxy which is optionally partially or fully halogenated, phenyloxy, naphthyloxy, heteroaryloxy or heterocyclicoxy wherein the heteroaryl or heterocyclic moiety is as hereinabove described in this paragraph, nitro, mono- or di-(C₁₋₃alkyl)amino, phenylamino, naphthylamino, heteroaryl or heterocyclic amino wherein the heteroaryl or heterocyclic moiety is as hereinabove described in this paragraph, NH₂C(O), mono- or di-(C₁₋₃alkyl)aminocarbonyl, C₁₋₄ alkyl-OC(O), C₁₋₅ alkyl-C(O)-C₁₋₄ alkyl, amino-C₁₋₅ alkyl and mono- or di-(C₁₋₃)alkylamino-C₁₋₅ alkyl;
   cyclopropanyl, cyclobutanyl, cyclopentanyl, cyclohexanyl, cycloheptanyl, bicyclopentanyl, bicyclohexanyl and bicycloheptanyl, each being optionally partially or fully halogenated and optionally substituted with one to three C₁₋₃ alkyl groups;
   cyclopentenyl, cyclohexenyl, cyclohexadienyl, cycloheptenyl, cycloheptadienyl, bicyclohexenyl or bicycloheptenyl, each optionally substituted with one to three C₁₋₃ alkyl groups;
      and
   C₁₋₆ alkyl branched or unbranched and optionally partially or fully halogenated;
R₁₁ is chosen from hydrogen and C₁₋₄ branched or unbranched alkyl which may optionally be partially or fully halogenated;
each R₇, R₈, R₉, R₁₀, is independently chosen from:
   morpholine, piperidine, piperazine, imidazole and tetrazole;
wherein said method comprises:
reacting a compound of the formula(II) with a compound of the formula(III) under acid pH conditions, in a polar protic solvent under reflux for 5-16 hours, according to the scheme below: 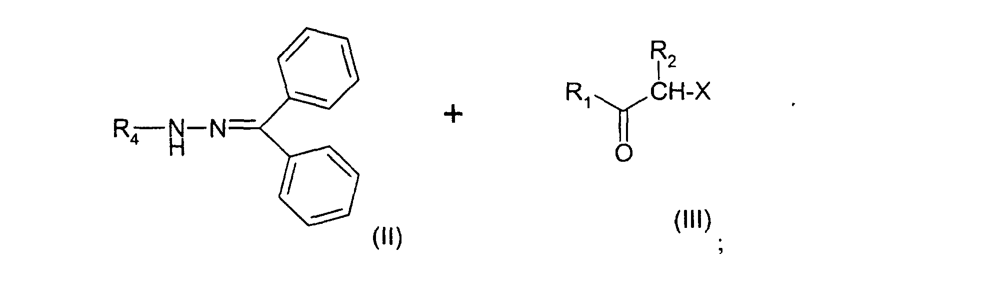
wherein X is chosen from -CN and -C(O)-R₃, wherein if X is CN then R₃ in the product formula (I) is amino; to form the product compound of the formula(I): 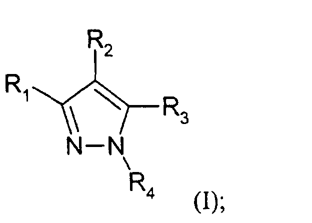 and subsequently isolating said product.

In another embodiment of the invention there is provided a process as described above and wherein R₂ is H;

In another embodiment of the invention there is provided a process as described immediately above and wherein:
the acid is chosen from HCl, AcOH, TFA and p-TsOH;
the solvent is a C₁-C₃ alcohol;
R₁ and R₃ are chosen from
   amine, C₁₋₁₀ alkyl, alkoxy, phenyl, naphthyl, pyridinyl, pyrimidinyl, pyrazinyl, pyridazinyl, pyrrolyl, imidazolyl, pyrazolyl, thienyl, furyl, isoxazolyl and isothiazolyl; each of the aforementioned being optionally substituted with one to three groups chosen from halogen, C₁₋₆alkyl and C₁₋₃ alkoxy; wherein when either R₁ or R₃ is amine the other is not amine;
   and
R₄ is chosen from:
   phenyl, naphthyl, pyridinyl, pyrimidinyl, pyrazinyl, pyrrolyl, imidazolyl and pyrazolyl, each of the aforementioned is optionally substituted with C₁₋₈ alkyl or C₁₋₆ branched or unbranched alkoxy each of which is optionally partially or fully halogenated.

In yet another embodiment, there is the process as described immediately above, and wherein
the acid is chosen from HCl and p-TsOH; the solvent is ethanol, the reflux time is 5-8 hours, R₃ is amino and
X is CN.

All terms as used herein in this specification, unless otherwise stated, shall be understood in their ordinary meaning as known in the art and be understood to be optionally substituted. For example, "alkoxy" is a alkyl with a terminal oxygen, such as methoxy, ethoxy and propoxy. All alkyl, alkenyl and alkynyl groups shall be understood as being branched or unbranched where structurally possible and unless otherwise specified. Other more specific definitions are as follows:

The term "aroyl" as used in the present specification shall be understood to mean "benzoyl" or "naphthoyl".

The term "heterocycle" refers to a stable nonaromatic 4-8 membered (but preferably, 5 or 6 membered) monocyclic or nonaromatic 8-11 membered bicyclic heterocycle radical which may be either saturated or unsaturated. Each heterocycle consists of carbon atoms and one or more, preferably from 1 to 4 heteroatoms chosen from nitrogen, oxygen and sulfur. The heterocycle may be attached by any atom of the cycle, which results in the creation of a stable structure. Examples of heterocycles include but are not limited to, oxetanyl, pyrrolidinyl, tetrahydrofuranyl, tetrahydrothiophenyl, piperidinyl, piperazinyl, morpholinyl, tetrahydropyranyl, dioxanyl, tetramethylene sulfonyl, tetramethylene sulfoxidyl, oxazolinyl, thiazolinyl, imidazolinyl, tertrahydropyridinyl, homopiperidinyl, pyrrolinyl, tetrahydropyrimidinyl, decahydroquinolinyl, decahydroisoquinolinyl, thiomorpholinyl, thiazolidinyl, dihydrooxazinyl, dihydropyranyl, oxocanyl, heptacanyl, thioxanyl and dithianyl.

The term "heteroaryl" shall be understood to mean an aromatic 5-8 membered monocyclic or 8-11 membered bicyclic ring containing 1-4 heteroatoms such as N,O and S. Examples of such heteroaryls include: pyridinyl, pyridonyl, quinolinyl, dihydroquinolinyl, tetrahydroquinoyl, isoquinolinyl, tetrahydroisoquinoyl, pyridazinyl, pyrimidinyl, pyrazinyl, benzimidazolyl, benzthiazolyl, benzoxazolyl, benzofuranyl, benzothiophenyl, benzpyrazolyl, dihydrobenzofuranyl, dihydrobenzothiophenyl, benzooxazolonyl, benzo[1,4]oxazin-3-onyl, benzodioxolyl, benzo[1,3]dioxol-2-onyl, tetrahydrobenzopyranyl, indolyl, indolinyl, indolonyl, indolinonyl and phthalimidyl.

The term "aryl" as used herein shall be understood to mean phenyl, tolyl or naphthyl.

Terms which are analogs of the above cyclic moieties such as aryloxy or heteroaryl amine shall be understood to mean an aryl, heteroaryl and heterocycle as defined above attached to it's respective functional group.

As used herein, "nitrogen" and "sulfur" include any oxidized form of nitrogen and sulfur and the quaternized form of any basic nitrogen.

The term "halogen" as used in the present specification shall be understood to mean bromine, chlorine, fluorine or iodine.

ETOH shall be understood to mean ethanol.

p-TsOH is para-toluenesulphonic acid.

TFA is trifluoroacetic acid.

AcOH is acetic acid.

DPPF is diphenylphosphinoferocene.

The method of the invention is directed to only making compounds which are contemplated to be 'chemically stable' as will be appreciated by those skilled in the art. For example, a compound which would have a 'dangling valency', or a 'carbanion' are not compounds contemplated by the invention.

The general reaction scheme describing this invention is illustrated below. A benzophenone hydrazone of formula (II) is reacted with a 1,3-bifunctional intermediate of formula (III) under acid pH conditions, in a polar protic solvent for about 5-16 hours, where X is a carbonyl bearing R₃ (-C(O)R₃), or a nitrile (-CN), in which case R₃ will be an amine (NH₂) in the product of formula (I). Regarding preferred reaction time, 5-8 hours is preferred where X is -C(O)R₃ and 8-16 hours where X is CN. R₁, R₂, R₃, R₄ and X are as defined hereinabove:

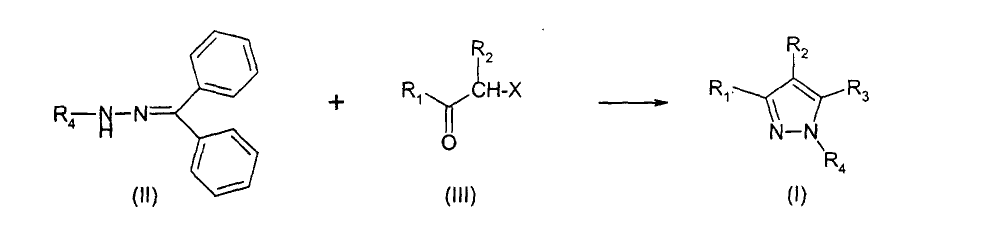

In one embodiment of the invention, the different acidic conditions can be obtained with acids chosen from: HCl, AcOH, TFA and p-TsOH. In yet another embodiment desirable yields are obtained by using p-TsOH or HCl in ethanol.

Schemes 1,2,3 and 4 represent specific aspects of the invention. These schemes are illustrative and, as recognized by one skilled in the art, particular reagents or conditions could be modified as needed for individual compounds without undue experimentation. Starting materials used in the schemes below are either commercially available or easily prepared from commercially available materials by those skilled in the art. Isolation and purification methods for particular compounds will be apparent to those of ordinary skill, a non-limiting example of which is provided in Example 1 below.

Aryl hydrazones **3, 4** and **11** were prepared by Pd-catalyzed cross-coupling of the corresponding aryl bromide with benzophenone hydrazone, following the recently reported procedure by Hartwig.^{3b} The hydrazones were obtained in 85-99% yields. See Scheme 1 below. 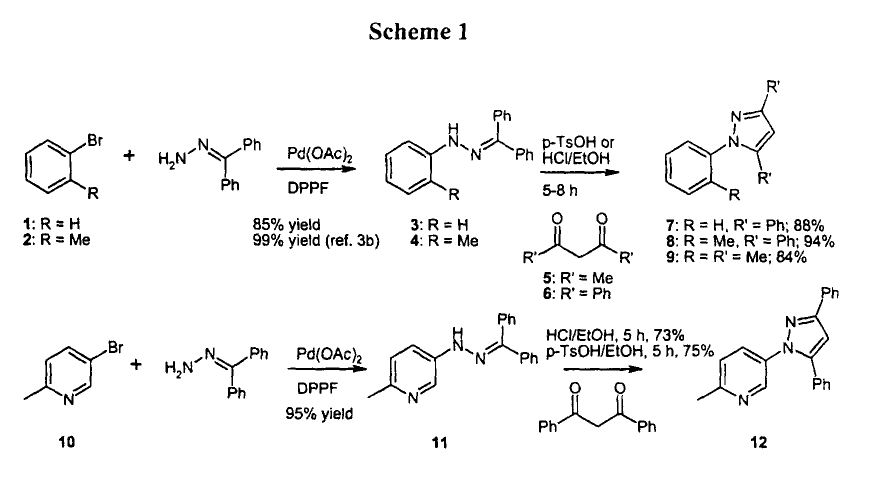 The synthesis of pyrazoles is accomplished by refluxing hydrazones **3, 4** and **11** (shown below) with symmetrical 1,3-diketones **5** and **6** in ethanol under acidic conditions. Pyrazoles **8**, **9** and **12** were prepared in 75-94% isolated yields using p-TsOH. Similar yields were obtained in preparing pyrazoles **7** and **12** under HCl/EtOH conditions.⁵

The results with symmetrical diketones prompted examination of the regioselective synthesis of unsymmetrical pyrazoles or pyrazole-related structures from aryl hydrazones **3** and **4**. As illustrated in Scheme 2 treatment of **3** with ethyl acetoacetate, under p-TsOH/EtOH conditions, has provided pyrazoles **14** and pyrazolone **15** in 3:1 ratio respectively and 52% isolated yield. See Example 1. Interestingly, replacing p-TsOH with HCl provided **14a** and **15a** in 1:1 ratio and 58% yield. The stability of **14a** and **15a** under the p-TsOH and HCl reaction conditions was examined and no interconversion was detected in both compounds. On the other hand, a single product (14b) was formed in 41% yield upon treatment of **4** with p-TsOH and a 4:1 ratio of **14b:15b** was obtained in 38% yield under the HCl conditions.

Synthesis of pyrazolones **17** was accomplished by treatment of **3** or **4** with ethyl malonyl chloride in refluxing dioxane, affording after 10 min the corresponding compound **16** in 80-83% isolated yield. Subsequent cyclization of **16** in p-TsOH/EtOH afforded, after 1.5 h, pyrazolones **17** in 68-70% yield. The ¹H-NMR of compound **17a** is in full agreement with previously reported data.⁵

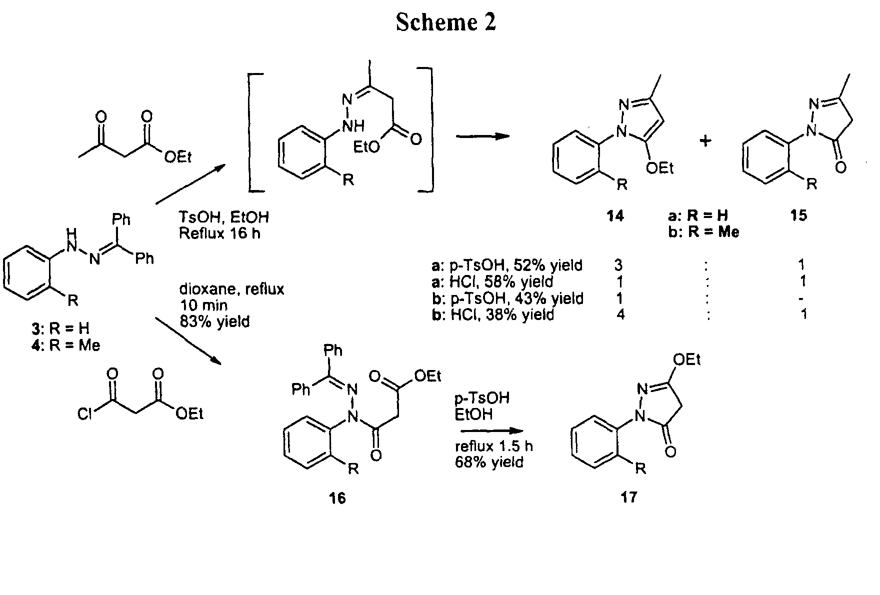

Preparation of pyrazole amines was expected to be possible by treating hydrazones with cyanoketone **18** under acidic conditions as illustrated in Scheme 3. Similar selectivity in the transhydrazonation to that obtained with β-ketoesters should provide pyrazole amines of type **19**. Treatment of aryl hydrazones **3** and **4** with **18** afforded single products **19** and **20** respectively in 80% isolated yields. The structure of **19** was confirmed by its preparation from hydrazine **21** with cyanoketone **18** under similar reaction conditions. The utility of the cross coupling-pyrazole formation sequence was further demonstrated in the synthesis of heteroaryl pyrazole **22** in 61% yield.

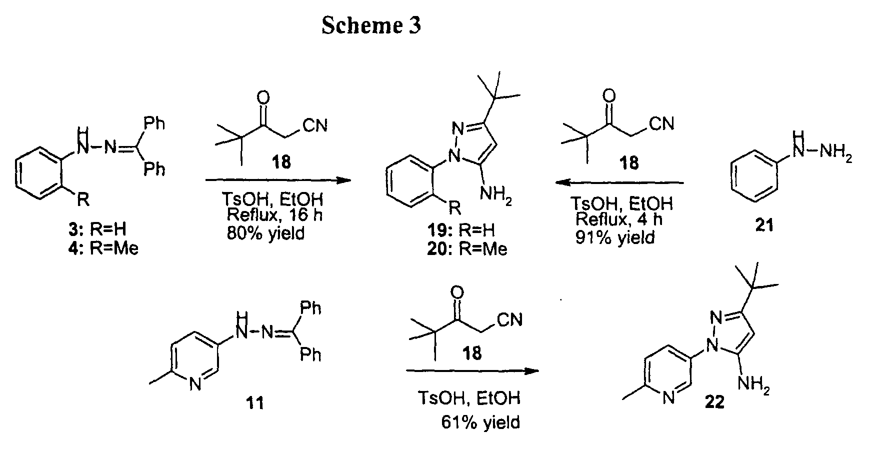

In order to examine the regioselectivity of pyrazole formation with unsymmetrical diketones, hydrazone **3** was treated with diketone **23** under conditions according to the invention. See Scheme 4 and Example 1. A mixture of isomers **24** and **25** was obtained in 7:1 ratio respectively in 82% total yield. It should be noted that 19:1 ratio of **24** vs. **25** respectively, was reported⁶ on their formation from hydrazine **21** and diketone **23.** High regioselectivity was expected in the pyrazole formation of diketone **26.** Indeed, single products **27** and **28** were obtained upon its reaction under the p- TsOH/EtOH conditions with hydrazones **4** and **11** in 84% and 88% yields respectively. ⁷

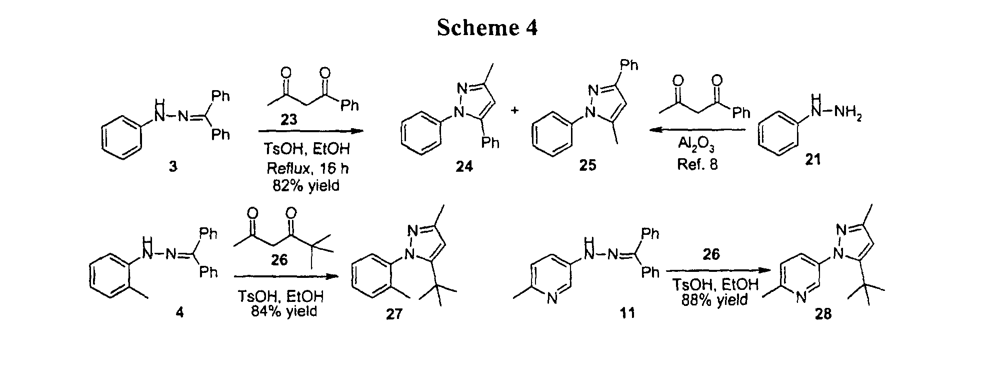

In order that this invention be more fully understood, the following examples 1(a) and (b) are set forth. These examples are for the purpose of illustrating embodiments of this invention, and are not to be construed as limiting the scope of the invention in any way.

### EXAMPLE 1

1(a) with p-TsOH/EtOH: A solution of the benzophenone hydrazone (1.75 mmol), p-TsOH (1.0 g) and the bi-functional substrate (2.63 mmol) in EtOH (10 mL) was refluxed for a period of 8-16 h. The reaction mixture was cooled to RT, then NaHCO₃ saturated solution (10 mL) and EtOAc (10 mL) were added. The layers were separated, and the aqueous layer washed with EtOAc. The combined organics dried (Na₂SO₄), concentrated then purified by column chromatography.

1(b) with HCl/EtOH: The reactions were carried out in a saturated solution of HCI in EtOH with a similar ratio of reactants and concentration as described in (a). Excess saturated NaHCO₃ was added to ensure complete neutralization of the HCl.

All new compounds were characterized by full spectroscopic data, yields refer to chromatographed materials with purity of > 95%. Selected ¹H-NMR data from **14a:** δ 5.47 (1H, s), 4.12 (2H, q), 2.28 (3H, s), 1.43 (3H, t); **14a** (literature^{a}): δ 5.50 (1H, s), 4.14 (2H, q), 2.26 (3H, s), 1.41 (3H, t); **14b:** δ 5.44 (1H, s), 4.07 (2H, q), 2.26 (3H, s), 1.33 (3H, t). (a) Katritzky, A. R.; Main, F. W. *Tetrahedron* **1964,** *20,* 299; ¹H-NMR of **15a** found in full agreement with reported data: DeRuiter, J.; Carter, D. A.; Arledge, W. S.; Sullivan, P. J. *J. Heterocyclic Chem.* **1987,** *24,* 149.

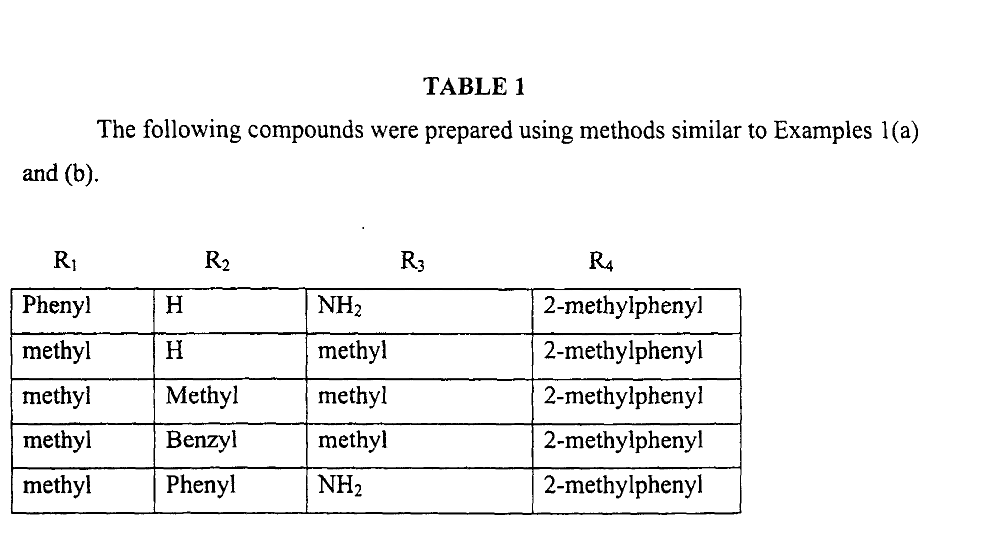

### References and Notes

1. (a) Makino, K.; Kim, H. S.; Kurasawa, *Y. J. Heterocyclic Chem.* **1998,** 35, 489; (b) Elguero, J. *Compr. Heterocycl. Chem. II* **1996,** *3*, 1.
2. For reviews on the synthesis of pyrazoles and pyrazole related structures see: ref. 1 and (a) Takagi, K.; Huber-Habart, M. *J. Heterocyclic Chem.* **1996,** 33, 1003; (b) El-Rayyes, N. R.; Al-Awadi, N. A. *Synthesis* **1985,** 1028; (c) Sammes, M. P.; Katritzky, A. R. *Advances in Heterocyclic Chemistry, Vol 34,* Academic Press, **1983**; (d) Behr, L. C.; Fusco, R.; Jarboe, C. H. *The Chemistry of Heterocyclic Compounds,* Weissberger, A., ed., Interscience Publishers, John Wiley and Sons, **1967.**
3. (a) Wagaw, S.; Yang, H. B.; Buchwald, S. L. *J*. *Am. Chem. Soc.* **1998,** *120*, 6621; (b) Hartwig, J. F. *Angew. Chew., Int. Ed.* **1998,** 37, 2090.
4. For palladium catalyzed coupling of t-butylcarbazate with *activated* aryl bromides see: Wang, Z.; Skerlj, R. T.; Bridger, G. J. *Tet. Lett.* **1999,** *40,* 3543.
5. Selected ¹H-NMR data from **17a:** δ 4.35 (2H, q), 3.48 (1H, s); **17a** (literature^{7a}): δ 4.34 (2H, q), 3.47 (1H, s); **17b:** δ 4.27 (2H, q), 3.46 (1H, s); (a) Molinari, A.; Oliva, A. *J. Heterocyclic Chem.* **1996,** 33, 479.
6. Texier-Boullet, F.; Klein, B.; Hamelin, J. *Synthesis* **1986**, 409.
7. The regioselectivity in structures **27** and **28** were confirmed by the NOE between the t-butyl with the N-aryl substituent, determined by NOESY.

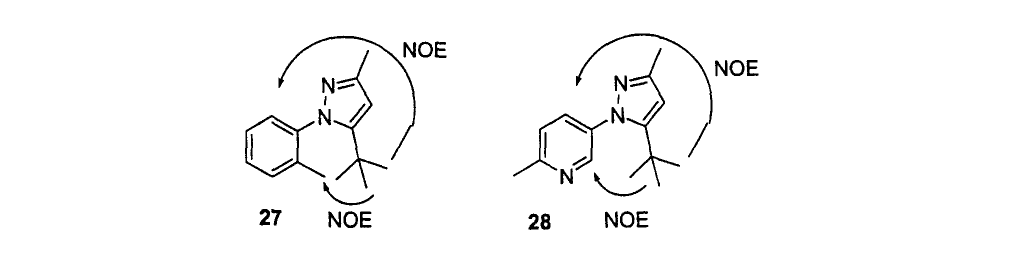

All references cited in this application are incorporated herein by reference in their entirety.

## Claims

1. A method of making a pyrazole compound of the formula(I): 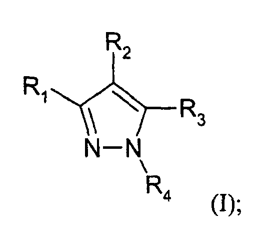 wherein R₁, R₂, R₃ and R₄ are defined as follows:
each R₁ and R₃ are independently chosen from:
amino and C₁₋₁₀ alkyl optionally partially or fully halogenated and optionally substituted with one to three C₃₋₁₀ cycloalkanyl, C₁₋₆alkoxy, phenyl, naphthyl, pyridinyl, pyrimidinyl, pyrazinyl, pyridazinyl, pyrrolyl, imidazolyl, pyrazolyl, thienyl, furyl, isoxazolyl or isothiazolyl; each of the aforementioned being optionally substituted with one to five groups chosen from halogen, C₁₋₆ alkyl which is optionally partially or fully halogenated, C₃₋₈ cycloalkanyl, C₅₋₈ cycloalkenyl and C₁₋₃ alkoxy which is optionally partially or fully halogenated; wherein both R₁ and R₃ cannot simultaneously be amino;
R₂ is chosen from:
hydrogen, C₁₋₆ branched or unbranched alkyl optionally partially or fully halogenated and aryl optionally partially or fully halogenated;
R₄ is chosen from:
phenyl, naphthyl, morpholinyl, pyridinyl, pyrimidinyl, pyrazinyl, pyridazinyl, pyrrolyl, pyrrolidinyl, imidazolyl, pyrazolyl, thiazolyl, oxazoyl, triazolyl, tetrazolyl, thienyl, furyl, tetrahydrofuryl, isoxazolyl, isothiazolyl, quinolinyl, isoquinolinyl, indolyl, benzimidazolyl, benzofuranyl, benzoxazolyl, benzisoxazolyl, benzpyrazolyl, benzothiofuranyl, cinnolinyl, pterindinyl, phthalazinyl, naphthypyridinyl, quinoxalinyl, quinazolinyl, purinyl and indazolyl, each of the aforementioned is optionally substituted with one to three phenyl, naphthyl, heterocycle or heteroaryl as hereinabove described in this paragraph, C₁₋₆ branched or unbranched alkyl which is optionally partially or fully halogenated, cyclopropanyl, cyclobutanyl, cyclopentanyl, cyclohexanyl, cycloheptanyl, bicyclopentanyl, bicyclohexanyl, bicycloheptanyl, phenyl C₁₋₅ alkyl, naphthyl C₁₋₅ alkyl, halogen, hydroxy, oxo, nitrile, C₁₋₃ alkoxy optionally partially or fully halogenated, phenyloxy, naphthyloxy, heteroaryloxy or heterocyclicoxy wherein the heterocyclic or heteroaryl moiety is as hereinabove described in this paragraph, nitro, phenylamino, naphthylamino, heteroaryl or heterocyclic amino wherein the heteroaryl or heterocyclic moiety is as hereinabove described in this paragraph, NH₂C(O), a mono- or di-(C₁₋₃alkyl) aminocarbonyl, C₁₋₅ alkyl-C(O)-C₁₋₄ alkyl, amino-C₁₋₅ alkyl, mono- or di-(C₁₋₃alkyl)amino-C₁₋₅ alkyl, amino-S(O)₂, di-(C₁₋₃alkyl)amino-S(O)₂, R₇-C₁₋₅alkyl, R₈-C₁₋₅ alkoxy, R₉-C(O)-C₁₋₅ alkyl, R₁₀-C₁₋₅ alkyl(R₁₁)N or carboxy-mono- or di-(C₁₋₅alkyl)-amino;
a fused aryl chosen from benzocyclobutanyl, indanyl, indenyl, dihydronaphthyl, tetrahydronaphthyl, benzocycloheptanyl and benzocycloheptenyl, or a fused heteroaryl chosen from cyclopentenopyridinyl, cyclohexanopyridinyl, cyclopentanopyrimidinyl, cyclohexanopyrimidinyl, cyclopentanopyrazinyl, cyclohexanopyrazinyl, cyclopentanopyridazinyl, cyclohexanopyridazinyl, cyclopentanoquinolinyl, cyclohexanoquinolinyl, cyclopentanoisoquinolinyl, cyclohexanoisoquinolinyl, cyclopentanoindolyl, cyclohexanoindolyl, cyclopentanobenzimidazolyl, cyclohexanobenzimidazolyl, cyclopentanobenzoxazolyl, cyclohexanobenzoxazolyl, cyclopentanoimidazolyl, cyclohexanoimidazolyl, cyclopentanothienyl and cyclohexanothienyl; wherein the fused aryl or fused heteroaryl ring is independently substituted with zero to three phenyl, naphthyl, pyridinyl, pyrimidinyl, pyrazinyl, pyridazinyl, pyrrolyl, imidazolyl, pyrazolyl, thienyl, furyl, isoxazolyl, isothiazolyl, C₁₋₆ alkyl which is optionally partially or fully halogenated, halogen, nitrile, C₁₋₃ alkoxy which is optionally partially or fully halogenated, phenyloxy, naphthyloxy, heteroaryloxy or heterocyclicoxy wherein the heteroaryl or heterocyclic moiety is as hereinabove described in this paragraph, nitro, mono- or di-(C₁₋₃alkyl)amino, phenylamino, naphthylamino, heteroaryl or heterocyclic amino wherein the heteroaryl or heterocyclic moiety is as hereinabove described in this paragraph, NH₂C(O), mono- or di-(C₁₋₃alkyl)aminocarbonyl, C₁₋₄ alkyl-OC(O), C₁₋₅ alkyl-C(O)-C₁₋₄ alkyl, amino-C₁₋₅ alkyl and mono- or di-(C₁₋₃)alkylamino-C₁₋₅ alkyl;
cyclopropanyl, cyclobutanyl, cyclopentanyl, cyclohexanyl, cycloheptanyl, bicyclopentanyl, bicyclohexanyl and bicycloheptanyl, each being optionally partially or fully halogenated and optionally substituted with one to three C₁₋₃ alkyl groups;
cyclopentenyl, cyclohexenyl, cyclohexadienyl, cycloheptenyl, cycloheptadienyl, bicyclohexenyl and bicycloheptenyl, each optionally substituted with one to three C₁₋₃ alkyl groups;
and
C₁₋₆ alkyl branched or unbranched and optionally partially or fully halogenated;
R₁₁ is chosen from hydrogen and C₁₋₄ branched or unbranched alkyl which may optionally be partially or fully halogenated;
each R₇, R₈, R₉, R₁₀, is independently chosen from:
morpholine, piperidine, piperazine, imidazole and tetrazole;
wherein said method comprises:
reacting a compound of the formula(II) with a compound of the formula(III) under acid pH conditions, in a polar protic solvent under reflux for 5-16 hours, according to the scheme below: 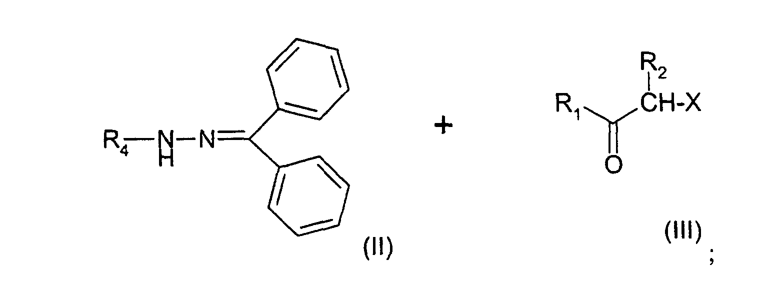
wherein X is chosen from -CN and -C(O)-R₃, wherein if X is CN then R₃ in the product formula (I) is amino; to form the product compound of the formula(I) 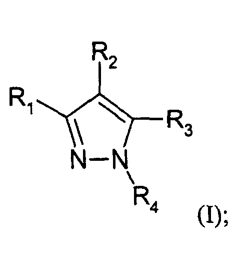 and subsequently isolating said product.

2. The process according to claim 1, wherein
R₂ is hydrogen.

3. The process according to claim 2, wherein
the acid is chosen from HCl, AcOH, TFA and p-TsOH;
the solvent is a C₁-C₃ alcohol;
R₁ and R₃ are chosen from
amino, C₁₋₁₀ alkyl, alkoxy, phenyl, naphthyl, pyridinyl, pyrimidinyl, pyrazinyl, pyridazinyl, pyrrolyl, imidazolyl, pyrazolyl, thienyl, furyl, isoxazolyl and isothiazolyl; each of the aforementioned being optionally substituted with one to three groups chosen from halogen, C₁₋₆alkyl and C₁₋₃ alkoxy; wherein when either R₁ or R₃ is amine the other is not amino;
and
R₄ is chosen from:
phenyl, naphthyl, pyridinyl, pyrimidinyl, pyrazinyl, pyrrolyl, imidazolyl and pyrazolyl, each of the aforementioned is optionally substituted with C₁₋₈ alkyl or C₁₋₆ branched or unbranched alkoxy each of which is optionally partially or fully halogenated.

4. The process according to claim 3 wherein:
the acid is chosen from HCl and p-TsOH;
the solvent is ethanol,
the reflux time is 5-8 hours;
R₃ is amino and
X is CN.

## Patentansprüche

1. Verfahren zur Herstellung einer Pyrazol-Verbindung der Formel (I): 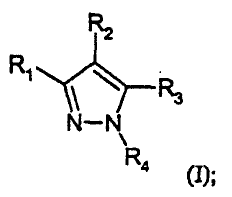 worin R₁, R₂, R₃ und R₄ folgendermaßen definiert sind:
R₁ und R₃ sind jeweils unabhängig ausgewählt aus:
Amino und C₁₋₁₀-Alkyl, gegebenenfalls teilweise oder vollständig halogeniert und gegebenenfalls substituiert mit 1 bis 3 C₃₋₁₀-Cycloalkanyl, C₁₋₆-Alkoxy, Phenyl, Naphthyl, Pyridinyl, Pyrimidinyl, Pyrazinyl, Pyridazinyl, Pyrrolyl, Imidazolyl, Pyrazolyl, Thienyl, Furyl, Isoxazolyl oder Isothiazolyl; wobei die vorstehend Genannten jeweils gegebenenfalls substituiert sind mit 1 bis 5 Gruppen, ausgewählt aus Halogen, C₁₋₆-Alkyl, das gegebenenfalls teilweise oder vollständig halogeniert ist, C₃₋₈-Cycloalkanyl, C₅₋₈-Cycloalkenyl und C₁₋₃-Alkoxy, das gegebenenfalls teilweise oder vollständig halogeniert ist; wobei R₁ und R₃ beide nicht gleichzeitig Amino sein können;
R₂ ist ausgewählt aus:
Wasserstoff, verzweigtem oder unverzweigtem C₁₋₆-Alkyl, gegebenenfalls teilweise oder vollständig halogeniert, und Aryl, gegebenenfalls teilweise oder vollständig halogeniert;
R₄ ist ausgewählt aus:
Phenyl, Naphthyl, Morpholinyl, Pyridinyl, Pyrimidinyl, Pyrazinyl, Pyridazinyl, Pyrrolyl, Pyrrolidinyl, Imidazolyl, Pyrazolyl, Thiazolyl, Oxazoyl, Triazolyl, Tetrazolyl, Thienyl, Furyl, Tetrahydrofuryl, Isoxazolyl, Isothiazolyl, Chinolinyl, Isochinolinyl, Indolyl, Benzimidazolyl, Benzofuranyl, Benzoxazolyl, Benzisoxazolyl, Benzpyrazolyl, Benzothiofuranyl, Cinnolinyl, Pteridinyl, Phthalazinyl, Naphthypyridinyl, Chinoxalinyl, Chinazolinyl, Purinyl und Indazolyl, wobei die vorstehend Genannten jeweils gegebenenfalls substituiert sind mit 1 bis 3 Phenyl, Naphthyl, Heterocyclo oder Heteroaryl, wie hier vorstehend in diesem Abschnitt beschrieben, verzweigtem oder unverzweigtem C₁₋₆-Alkyl, das gegebenenfalls teilweise oder vollständig halogeniert ist, Cyclopropanyl, Cyclobutanyl, Cyclopentanyl, Cyclohexanyl, Cycloheptanyl, Bicyclopentanyl, Bicyclohexanyl, Bicycloheptanyl, Phenyl-C₁₋₅-alkyl, Naphthyl-C₁₋₅-alkyl, Halogen, Hydroxy, Oxo, Nitril, C₁₋₃-Alkoxy, gegebenenfalls teilweise oder vollständig halogeniert, Phenyloxy, Naphthyloxy, Heteroaryloxy oder Heterocycloxy, wobei die heterocyclische Gruppe oder die Heteroarylgruppe wie hier vorstehend in diesem Abschnitt beschrieben ist, Nitro, Phenylamino, Naphthylamino, Heteroarylamino oder heterocyclischem Amino, worin die Heteroarylgruppe oder die heterocyclische Gruppe wie hier vorstehend in diesem Abschnitt beschrieben ist, NH₂C(O), einem Mono- oder Di-(C₁₋₃-alkyl)-aminocarbonyl, C₁₋₅-Alkyl-C(O)-C₁₋₄-alkyl, Amino-C₁₋₅-alkyl, Mono- oder Di-(C₁₋₃-alkyl)amino-C₁₋₅-alkyl, Amino-S(O)₂, Di-(C₁₋₃-alkyl)amino-S(O)₂, R₇-C₁₋₅-Alkyl, R₈-C₁₋₅-Alkoxy, R₉-C(O)-C₁₋₅-Alkyl, R₁₀-C₁₋₅-Alkyl(R₁₁)N oder Carboxy- mono- oder di-(C₁₋₅-alkyl)amino;
einem kondensierten Aryl, ausgewählt aus Benzocyclobutanyl, Indanyl, Indenyl, Dihydronaphthyl, Tetrahydronaphthyl, Benzocycloheptanyl und Benzocycloheptenyl, oder einem kondensierten Heteroaryl, ausgewählt aus Cyclopentenopyridinyl, Cyclohexanopyridinyl, Cyclopentanopyrimidinyl, Cyclohexanopyrimidinyl, Cyclopentanopyrazinyl, Cyclohexanopyrazinyl, Cyclopentanopyridazinyl, Cyclohexanopyridazinyl, Cyclopentanochinolinyl, Cyclohexanochinolinyl, Cyclopentanoisochinolinyl, Cyclohexanoisochinolinyl, Cyclopentanoindolyl, Cyclohexanoindolyl, Cyclopentanobenzimidazolyl, Cyclohexanobenzimidazolyl, Cyclopentanobenzoxazolyl, Cyclohexanobenzoxazolyl, Cyclopentanoimidazolyl, Cyclohexanoimidazolyl, Cyclopentanothienyl und Cyclohexanothienyl, wobei der kondensierte Aryl- oder kondensierte Heteroarylring unabhängig substituiert ist mit 0 bis 3 Phenyl, Naphthyl, Pyridinyl, Pyrimidinyl, Pyrazinyl, Pyridazinyl, Pyrrolyl, Imidazolyl, Pyrazolyl, Thienyl, Furyl, Isoxazolyl, Isothiazolyl, C₁₋₆-Alkyl, das gegebenenfalls teilweise oder vollständig halogeniert ist, Halogen, Nitril, C₁₋₃-Alkoxy, das gegebenenfalls teilweise oder vollständig halogeniert ist, Phenyloxy, Naphthyloxy, Heteroaryloxy oder Heterocycloxy, worin die Heteroarylgruppe oder die heterocyclische Gruppe wie hier vorstehend in diesem Abschnitt beschrieben ist, Nitro, Mono- oder Di-(C₁₋₃-alkyl)amino, Phenylamino, Naphthylamino, Heteroarylamino oder heterocyclischem Amino, worin die Heteroarylgruppe oder die heterocyclische Gruppe wie hier vorstehend in diesem Abschnitt beschrieben ist, NH₂C(O), Mono- oder Di-(C₁₋₃-alkyl)aminocarbonyl, C₁₋₄-Alkyl-OC(O), C₁₋₅-Alkyl-C(O)-C₁₋₄-alkyl, Amino-C₁₋₅-alkyl und Mono- oder Di-(C₁₋₃)alkylamino-C₁₋₅-alkyl;
Cyclopropanyl, Cyclobutanyl, Cyclopentanyl, Cyclohexanyl, Cycloheptanyl, Bicyclopentanyl, Bicyclohexanyl und Bicycloheptanyl, die jeweils gegebenenfalls teilweise oder vollständig halogeniert sind und gegebenenfalls mit 1 bis 3 C₁₋₃-Alkylgruppen substituiert sind;
Cyclopentenyl, Cyclohexenyl, Cyclohexadienyl, Cycloheptenyl, Cycloheptadienyl, Bicyclohexenyl und Bicycloheptenyl, die jeweils gegebenenfalls mit 1 bis 3 C₁₋₃-Alkylgruppen substituiert sind;
und
verzweigtem oder unverzweigtem C₁₋₆-Alkyl, das gegebenenfalls teilweise oder vollständig halogeniert ist;
wobei R₁₁ ausgewählt ist aus Wasserstoff und verzweigtem oder unverzweigtem C₁₋₄-Alkyl, das gegebenenfalls teilweise oder vollständig halogeniert sein kann;
R₇, R₈, R₉, R₁₀ jeweils unabhängig ausgewählt sind aus:
Morpholin, Piperidin, Piperazin, Imidazol und Tetrazol;
wobei das Verfahren umfasst:
das Umsetzen einer Verbindung der Formel (II) mit einer Verbindung der Formel (III) unter sauren pH-Bedingungen in einem polaren protischen Lösungsmittel unter Rückfluss für 5 bis 16 h nach dem nachstehenden Schema: 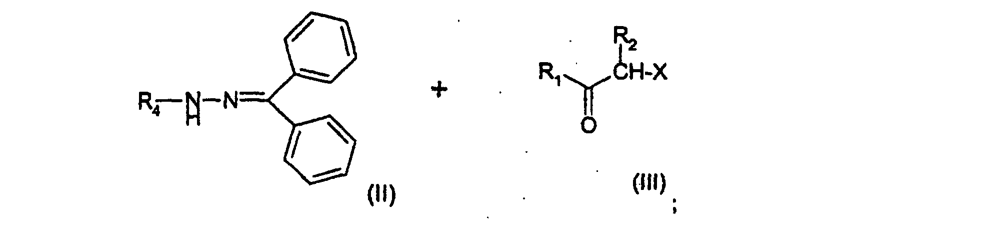
worin X ausgewählt ist aus -CN und -C(O)-R₃, wobei wenn X CN ist, R₃ in der Produktformel (I) Amino ist;
um die Produktverbindung der Formel (I) zu bilden: 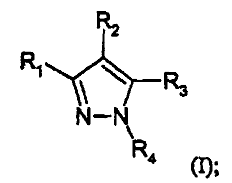 und das anschließende Isolieren des Produkts.

2. Verfahren nach Anspruch 1, worin R₂ Wasserstoff ist.

3. Verfahren nach Anspruch 2, worin die Säure ausgewählt ist aus HCl, AcOH, TFA und p-TsOH, das Lösungsmittel ein C₁-C₃-Alkohol ist; R₁ und R₃ ausgewählt sind aus Amino, C₁₋₁₀-Alkyl, Alkoxy, Phenyl, Naphthyl, Pyridinyl, Pyrimidinyl, Pyrazinyl, Pyridazinyl, Pyrrolyl, imidazolyl, Pyrazolyl, Thienyl, Furyl, Isoxazolyl und Isothiazolyl; wobei die vorstehend genannten jeweils gegebenenfalls substituiert sind mit 1 bis 3 Gruppen ausgewählt aus Halogen, C₁₋₆-Alkyl und C₁₋₃-Alkoxy; wobei wenn entweder R₁ oder R₃ Amin ist, das andere nicht Amino ist; und R₄ ausgewählt ist aus Phenyl, Naphthyl, Pyridinyl, Pyrimidinyl, Pyrazinyl, Pyrrolyl, Imidazolyl und Pyrazolyl, wobei die vorstehend genannten jeweils gegebenenfalls mit C₁₋₈-Alkyl oder verzweigtem oder unverzweigtem C₁₋₆-Alkoxy substituiert sind, die jeweils gegebenenfalls teilweise oder vollständig halogeniert sind.

4. Verfahren nach Anspruch 3, worin die Säure aus HCI und p-TsOH ausgewählt ist, das Lösungsmittel Ethanol ist, die Refluxzeit 5 bis 8 Stunden beträgt; R₃ Amino ist und X CN ist.

## Revendications

1. Procédé de production d'un composé du pyrazole de formule (I) : 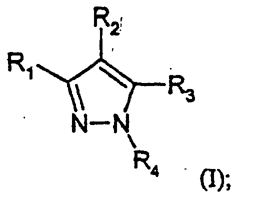 où R₁, R₂, R₃ et R₄ sont définis de la manière suivante :
R₁ et R₃ sont choisis chacun indépendamment parmi :
amino et alkyle en C₁₋₁₀ éventuellement partiellement ou totalement halogéné et éventuellement substitué par un à trois cycloalcanyle en C₃₋₁₀, alcoxy en C₁₋₆, phényle, napthyle, pyridinyle, pyrimidinyle, pyrazinyle, pyridazinyle, pyrrolyle, imidazolyle, pyrazolyle, thiényle, furyle, isoxazolyle ou isothiazolyle ; chacun des précédents étant éventuellement substitué par un à cinq groupes choisis parmi halogène, alkyle en C₁₋₆ qui est éventuellement partiellement ou totalement halogéné, cycloalcanyle en C₃₋₈, cycloalcényle en C₅₋₈ et alcoxy en C₁₋₃ qui est éventuellement partiellement ou totalement halogéné ; où R₁ et R₃ ne peuvent pas être amino simultanément ;
R₂ est choisi parmi :
l'hydrogène, alkyle en C₁₋₆ ramifié ou non ramifié éventuellement partiellement ou totalement halogéné et aryle éventuellement partiellement ou totalement halogéné ;
R₄ est choisi parmi :
phényle, naphtyle, morpholinyle, pyridinyle, pyrimidinyle, pyrazinyle, pyridazinyle, pyrrolyle, pyrrolidinyle, imidazolyle, pyrazolyle, thiazolyle, oxazolyle, triazolyle, tétrazolyle, thiényle, furyle, tétrahydrofuryle, isoxazolyle, isothiazolyle, quinolinyle, isoquinolinyle, indolyle, benzimidazolyle, benzofuranyle, benzoxazolyle, benzisoxazolyle, benzopyrazolyle, benzothiofuranyle, cinnolinyle, ptéridinyle, phtalazinyle, naphtylpyridinyle, quinoxalinyle, quinazolinyle, purinyle et indazolyle, chacun des groupes mentionnés précédemment est éventuellement substitué par un à trois phényle, napthyle, hétérocycles ou hétéroaryles comme décrit ci-dessus dans ce paragraphe, alkyle en en C₁₋₆ ramifié ou non ramifié qui est éventuellement partiellement ou totalement halogéné, cyclopropanyle, cyclobutanyle, cyclopentanyle, cyclohexanyle, cycloheptanyle, bicyclopentanyle, bicyclohexanyle, bicycloheptanyle, phényl-alkyle en C₁₋₅, naphtyl-alkyle en C₁₋₅, halogène, hydroxyle, oxo, nitrile, alcoxy en C₁₋₃ éventuellement partiellement ou totalement halogéné, phényloxy, naphtyloxy, hétéroaryloxy ou hétérocyclo-oxy où l'entité hétérocyclique ou hétéroaryle est décrite comme précédemment dans ce paragraphe, nitro, phénylamino, naphtylamino, hétéroaryl- ou hétérocyclo-amino où l'entité hétéroaryle ou hétérocyclique est décrite comme précédemment dans ce paragraphe, NH₂C(O), un mono- ou di-(alkyle en C₁₋₃)aminocarbonyle, alkyle en C₁₋₅-C(O)-alkyle en en C₁₋₄, amino-alkyle en C₁₋₅, mono- ou di-(alkyle en C₁₋₃)amino-alkyle en C₁₋₅, amino-S(O)₂, di-(alkyle en C₁₋₃)amino-S(O)₂, R₇-alkyle en C₁₋₅, R₈-alcoxy en C₁₋₅, R₉-C(O)-alkyle en C₁₋₅, R₁₀-alkyle en C₁₋₅-(R₁₁)N ou carboxy-mono- ou di-(alkyle en C₁₋₅)amino ; un aryle condensé choisi parmi benzocyclobutanyle, indanyle, indényle, dihydronaphtyle, tétrahydronaphtyle, benzocycloheptanyle et benzocycloheptényle, ou un hétéroaryle condensé choisi parmi cyclopenténopyridinyle, cyclohexanopyridinyle, cyclopentanopyrimidinyle, cyclohexanopyrimidinyle, cyclopentanopyrazinyle, cyclohexanopyrazinyle, cyclopentanopyridazinyle, cyclohexanopyridazinyle, cyclopentanoquinolinyle, cyclohexanoquinolinyle, cyclopentanoisoquinolinyle, cyclohexanoisoquinolinyle, cyclopentanoindolyle, cyclohexanoindolyle, cyclopentanobenzimidazolyle, cyclohexanobenzimidazolyle, cyclopentanobenzoxazolyle, cyclohexanobenzoxazolyle, cyclopentanoimidazolyle, cyclohexanoimidazolyle; cyclopentanothiényle et cyclohexanothiényle ; où le cycle aryle condensé ou hétéroaryle condensé est substitué indépendamment par zéro à trois phényle, naphtyle, pyridinyle, pyrimidinyle, pyrazinyle, pyridazinyle, pyrrolyle, imidazolyle, pyrazolyle, thiényle, furyle, isoxazolyle, isothiazolyle, alkyle en C₁₋₆ qui est éventuellement partiellement ou totalement halogéné, halogène, nitrile, alcoxy en C₁₋₃ qui est éventuellement partiellement ou totalement halogéné, phényloxy, naphtyloxy, hétéroaryloxy ou hétérocyclo-oxy où l'entité hétéroaryle ou hétérocyclique est comme décrit précédemment dans ce paragraphe, nitro, mono- ou di- (alkyle en C₁₋₃)amino, phénylamino, naphtylamino, hétéroaryl- ou hétérocyclo-amino où l'entité hétéroaryle ou hétérocyclique est comme décrit ci-dessus dans ce paragraphe, NH₂C(O), mono- ou di-(alkyle en C₁₋₃)aminocarbonyle, alkyle en C₁₋₄-OC(O), alkyle en C₁₋₅-C(O)-alkyle en C₁₋₄, amino-alkyle en C₁₋₅ et mono- ou di-(alkyle en C₁₋₃)amino-alkyle en C₁₋₅ ;
cyclopropanyle, cyclobutanyle, cyclopentanyle, cyclohexanyle, cycloheptanyle, bicyclopentanyle, bicyclohexanyle et bicycloheptanyle, chacun étant éventuellement partiellement ou totalement halogéné et éventuellement substitué par un à trois groupes alkyle en C₁₋₃ ;
cyclopentényle, cyclohexényle, cyclohexadiényle, cycloheptényle, cycloheptadiényle, bicyclohexényle et bicycloheptényle, chacun éventuellement substitué par un à trois groupes alkyle en C₁₋₃ ; et
alkyle en C₁₋₆ ramifié ou non ramifié et éventuellement partiellement ou totalement halogéné ;
R₁₁ est choisi parmi l'hydrogène et alkyle en C₁₋₄ ramifié ou non ramifié qui peut éventuellement être partiellement ou totalement halogène ;
R₇, R₈, R₈, R₁₀ sont choisis chacun indépendamment parmi :
morpholine, pipéridine, pipérazine, imidazole et tétrazole ;
où ledit procédé comprend :
la réaction d'un composé de formule (II) avec un composé de formule (III) dans des conditions de pH acide, dans un solvant protique polaire à reflux pendant 5-16 heures, selon le schéma ci-dessous : 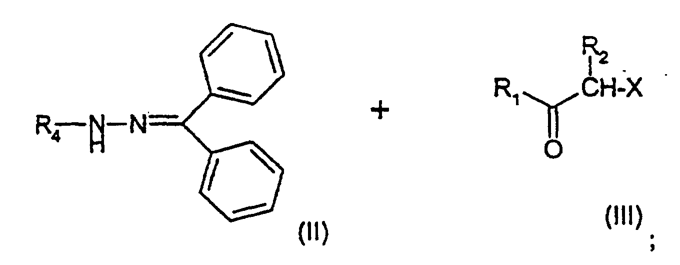
où X est choisi parmi -CN et -C(O)R₃, où, si X est CN, R₃ dans la formule de produit (I) est amino ;
pour former le composé de produit de formule (I) : 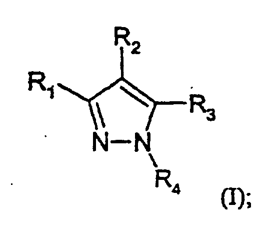 puis l'isolement dudit produit.

2. Procédé selon la revendication 1 où R₂ est l'hydrogène.

3. Procédé selon la revendication 2 où l'acide est choisi parmi HCl, AcOH, TFA et p-TsOH ;
le solvant est un alcool en C₁₋C₃ ;
R₁ et R₃ sont choisis parmi
amino, alkyle en C₁₋₁₀, alcoxy, phényle, napthyle, pyridinyle, pyrimidinyle, pyrazinyle, pyridazinyle, pyrrolyle, imidazolyle, pyrazolyle, thiényle, furyle, isoxazolyle et isothiazolyle ; chacun des précédents étant éventuellement substitué par un à trois groupes choisis parmi halogène, alkyle en C₁₋₆ et alcoxy en C₁₋₃; où, quand l'un ou l'autre parmi R₁ et R₃ est amine, l'autre n'est pas amino ;
et
R₄ est choisi parmi :
phényle, naphtyle, pyridinyle, pyrimidinyle, pyrazinyle, pyrrolyle, imidazolyle et pyrazolyle, chacun des groupes mentionnés précédemment est éventuellement substitué par alkyle en C₁₋₈ ou alcoxy en C₁₋₆ ramifié ou non ramifié qui sont chacun éventuellement partiellement ou totalement halogénés.

4. Procédé selon la revendication 3 où :
l'acide est choisi parmi HCl et p-TsOH ;
le solvant est l'éthanol,
la durée du reflux est 5-8 heures ;
R₃ est amino et
X est CN.
